# EUROPEAN PATENT APPLICATION

(11) **EP 2 995 238 A2**
(43) Date of publication of application: **16.03.2016**
(21) Application number: 15181688.1
(22) Date of filing: 20.08.2015
(51) Int. Cl.: A61B 1/00, G02B 23/24, G02B 21/22

(54) **COMPACT STEREOSCOPIC LENS SYSTEM FOR MEDICAL OR INDUSTRIAL IMAGING DEVICE**

(30) Priority: 22.08.2014 US 201414466283
(71) Applicant: Karl Storz Imaging Inc., Goleta, CA 93117 (US)
(72) Inventor: Duckett, George E., Goleta, CA 93117 (US)
(74) Representative: Witte, Weller & Partner Patentanwälte mbB

(57) **Abstract**

An imaging device, including an optical channel, a lens at a distal end of the optical channel, a differentiating element in the optical channel receiving light from the lens and differentiating the light into different wavelengths, a first sensor at a proximal end of the optical channel, a second sensor at the proximal end of the optical channel, the second sensor oriented substantially perpendicular to the first sensor, and a selection element between the first and second sensors receiving the differentiated light from the differentiating element via the optical channel and transmitting a first portion of the differentiated light to the first sensor and a second portion of the differentiated light to the second sensor.

## Description

### FIELD OF THE INVENTION

The invention relates to medical and industrial imaging devices, and more specifically to a stereoscopic lens system for medical and industrial imaging devices.

### BACKGROUND OF THE INVENTION

Imaging devices, including medical endoscopes and industrial borescopes, are known and widely used. An endoscope is a medical device for insertion into a body passageway or cavity that enables an operator to view and/or perform certain surgical procedures at a site inside a patient's body. Endoscopes may be either rigid or flexible, and generally include a long tubular member equipped with, for example, a system for transmitting images to the user, and in some cases, a working channel for a surgical instrument. The endoscope has a proximal end that remains external to the patient, from which the operator can view the site and/or manipulate a surgical instrument, and a distal end having an endoscope tip for insertion into the body cavity of the patient.

Borescopes generally have a similar design but may be used for visual inspection work in industrial settings. For example, a borescope may be used in non-destructive testing and inspections for defects and imperfections in machinery, such as aircraft engines, industrial gas turbines, and automotive engines.

Generally, these instruments employ some form of objective lens system, which focuses the image onto some form of image guide, such as a fiber optic bundle or relay lenses, thereby transmitting the images from inside a cavity to the user's eye located at the proximal end of the endoscope, or for subsequent display on a monitor and/or storage on an image capture device.

Imaging devices can provide two-dimensional (2D) imagery or, in some cases, three-dimensional (3D) imagery. 3D has the advantage of providing the surgeon or operator with depth perception during the procedure or inspection. However, creating an optical system for small diameter 3D imaging systems, while maintaining adequate quality, has been difficult. Further, conventional solutions are too large and not suitable for smaller imaging devices.

It is therefore desired to provide an improved imaging system with a sensor and lens arrangement that overcomes the drawbacks in the prior art.

### SUMMARY OF THE INVENTION

Accordingly, it is an object of the present invention to provide an imaging device and lens system which provides for stereo viewing with high resolution and/or increased resolution. It is a further object of the present invention to provide a lens system with a compact design for imaging devices having a small diameter, such as 4 mm or less.

These and other objectives are achieved by providing an imaging device, including an optical channel, a lens assembly, including one or more lenses, at a distal end of the optical channel, and differentiating element in the optical channel receiving light from the lens assembly and differentiating the light. The imaging device further includes a first sensor at a proximal end of the optical channel, a second sensor oriented substantially perpendicular to the first sensor at the proximal end of the optical channel, and selection element between the first and second sensors. The selection element receives the differentiated light from the differentiating element via the optical channel and transmits a first portion of the differentiated light to the first sensor and a second portion of the differentiated light to the second sensor.

In some embodiments, the imaging device further includes a second lens assembly at the distal end of the optical channel, wherein the light is received via the lens assembly and the second lens assembly and combined by the differentiating element into the optical channel. The differentiating element and selection element may be spectral filters or polarization elements, such as cube polarization beam splitters. In some embodiments, the differentiating element includes two polarizers in a pupil plane of the optical channel receiving the light from the lens.

The first sensor, the second sensor and the selection element may be comprised in a sensor package. The sensor package may be advantageously compact having, for example, a diagonal dimension of 3.7 millimeters or less or 3.0 millimeters or less. The imaging device may have a resolution of 600 x 600 or more, such as 694 x 694 or more.

Other objects of the present invention are achieved by provision of an imaging device, including a body, a shaft, and a lens system arranged within the shaft. The lens system includes at least one lens adjacent to a distal end of the shaft, first and second sensors arranged substantially perpendicular to one another in the shaft, and a selection element between the first and second sensors in the shaft.

In some embodiments, the lens system further includes a differentiating element in an optical path between the at least one lens and the selection element. The differentiating element receives light from the lens and transmits differentiated light via the optical path to the selection element. The at least one lens may be two lenses adjacent to the distal end of the shaft, each of the two lenses receiving light via the distal end of the imaging device.

Further provided is an imaging device, including a first lens receiving light via a distal end of the imaging device, a second lens receiving light via a distal end of the imaging device, a first sensor, and a second sensor. The second sensor is oriented substantially perpendicular to the first sensor. A differentiating element receives the light from the first lens and the light from the second lens, combines the light from the first lens with the light from the second lens into an optical channel, and transmits differentiated light via the optical channel. A selection element is situated between the first and second sensors receiving the differentiated light via the optical channel and transmitting a first portion of the differentiated light to the first sensor and a second portion of the differentiated light to the second sensor.

Other objects of the invention and its particular features and advantages will become more apparent from consideration of the following drawings and accompanying detailed description.

### BRIEF DESCRIPTION OF THE DRAWINGS

FIG. 1 is an imaging device according to an exemplary embodiment of the present invention.
FIG. 2 is exemplary embodiment of a lens system of the imaging device shown in FIG. 1.
FIG. 3 is exemplary embodiment of a lens system of the imaging device shown in FIG. 1.

### DETAILED DESCRIPTION OF THE INVENTION

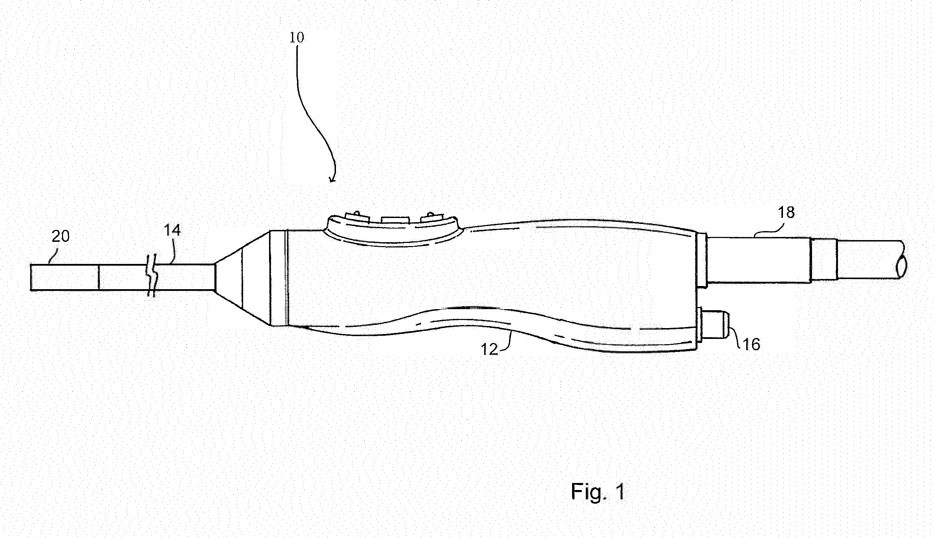

FIG. 1 illustrates an imaging device 10 according to the present invention. The imaging device 10 may be, for example, a medical endoscope or an industrial borescope. In a preferred embodiment, the imaging device is a stereoscopic 3D imaging device.

The imaging device 10 includes a body 12 and a shaft 14 connected to a distal end of the body 12. The shaft 14 may be flexible or rigid. The shaft 14 includes a distal tip 20 including a lens system. In the exemplary embodiment, the shaft 14 has an outer diameter of approximately 4 millimeters and an internal diameter of approximately 3.7 millimeters. In some embodiments, the imaging device 10 further includes a light post 16 connected to the body 12 receiving light for illumination via a light guide. The light is directed down the shaft 14 for illumination of a surgical area. In other embodiments, light is provided by LED internal to the body 12. The image device 10 may further include an imaging cable 18.

The lens system in the imaging device 10 includes at least one optical channel or path. Two sensors are provided at a proximal end of the optical channel. In a preferred embodiment, first and second sensors are arranged substantially perpendicular to one another. The lens system includes at least one light selection element. For example, a selection element may be arranged between the first and second sensors to receive light via the optical channel and transmit or reflect a first portion of differentiated light to the first sensor and a second portion of differentiated light to the second sensor. The selection element may be a spectral filter (e.g., a dichroic spectral filter) or a polarizer (e.g., a cube polarization beam splitter). The lens system may further include one or more light differentiating elements in the optical channel between at least one lens assembly or opening at the distal end of the optical channel and the selection element.

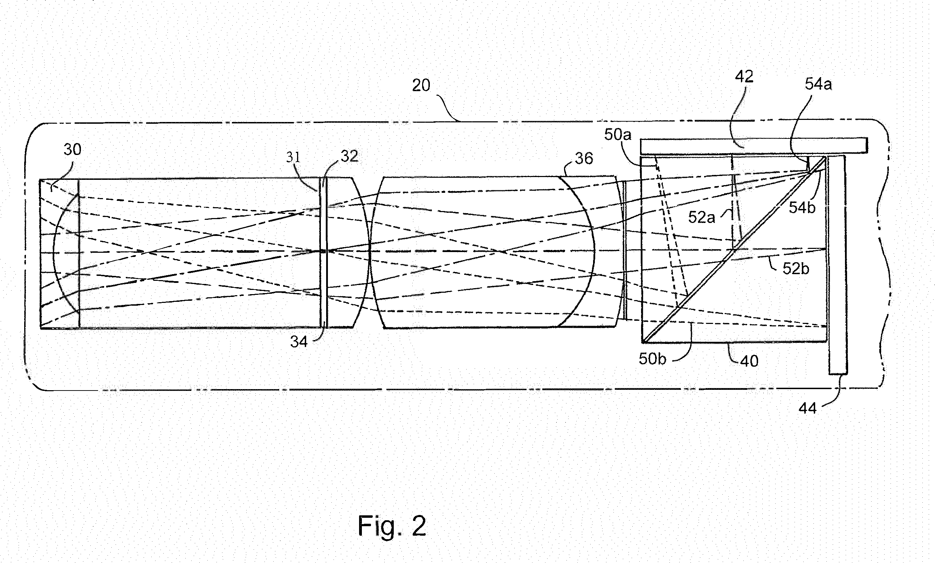

FIG. 2 illustrates one embodiment of the lens system including a single optical channel or path receiving light. The lens system may be provided in a portion of the shaft 14, such as in the distal tip 20. The lens system includes a lens assembly 30, including one or more lenses and defining a distal end of the optical channel. The lens system further includes at least one differentiating element 31. The differentiating element 31 may be a spectral filter differentiating between different wavelengths or colors of light received from the lens assembly 30 via the optical channel. The differentiating element 31 may alternatively be at least one polarizer, such as polarizers 32 and 34 located in the same pupil plane or cross-section of the optical channel so that the two halves of the pupil are polarized orthogonally. A first one of the polarizers or polarization elements 32 may be an s-pass (perpendicular) polarizer. A second one of the polarizers or polarization elements 34 may be a p-pass (parallel) polarizer. Light is transmitted through the optical channel or lens 36 to sensors and/or a sensor package.

The lens system further includes a sensor package having a selection element 40, a first sensor 42, and a second sensor 44. The selection element 40 may be a spectral filter (e.g., a dichroic spectral filter) filtering and/or reflecting different wavelengths or colors of light from the differentiating element 31 to each of the first and second sensors 42/44. Alternatively, the selection element 40 may be a polarizer, such as a cube polarization beam splitter, splitting light into beams of differing polarization, reflecting light from one half of the pupil (light 50a, 52a, 54a from polarizer 32) while transmitting light from the other half (light 50b, 52b, 54b from polarizer 34). Each half of the pupil is imaged on its own sensor 42/44 in the sensor package, creating two channels for 3D imaging. In particular, the sensor package includes a first sensor 42 receiving light 50a, 52a, and 54a and a second sensor 44 receiving light 50b, 52b, and 54b. The first sensor 42 and a second sensor 44 are oriented substantially perpendicular to, i.e., approximately ninety degrees from, one another in the sensor package.

The lens system of FIG. 2 advantageously provides a compact lens system. For example, the sensor package may be less than 3mm diagonal. The lens system also provides full sensor resolution of 694 x 694 or more.

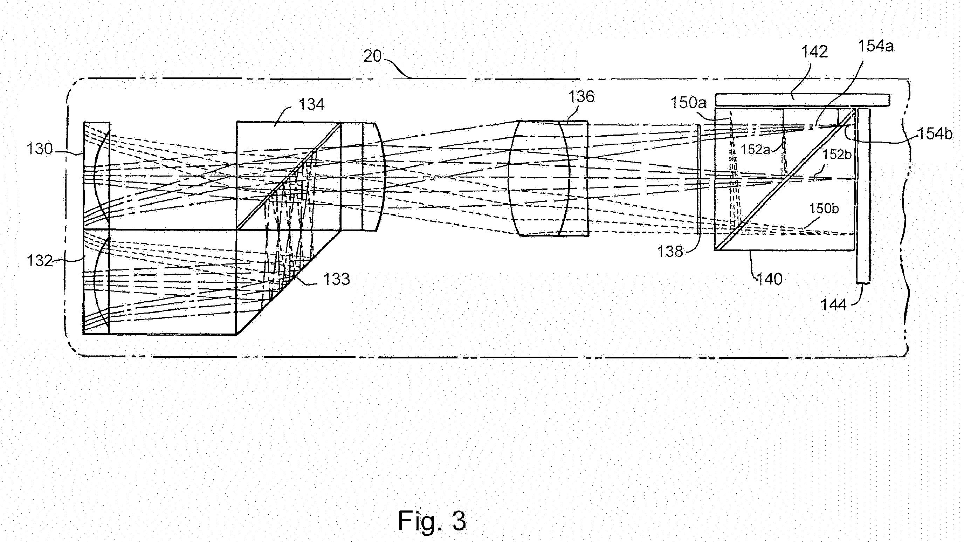

FIG. 3 illustrates another embodiment of the lens system including dual optical channels receiving light. The lens system may be provided in a portion of the shaft 14, such as in the distal tip 20. This embodiment is similar to the embodiment of FIG. 2 except the distal portion of the lens group is separated into two channels to increase the disparity. The lens system includes a lens assembly 130 and a lens assembly 132 defining distal ends of the two optical channels. The optical channels are combined and light simultaneously differentiated between different wavelengths by a differentiating element 134. The differentiating element 134 may be, for example, a spectral filter. Alternatively, the differentiating element 134 may be a polarizer, such as a cube polarization beam splitter, polarizing light from the lenses 130 and 132. In particular, the first cube polarization beam splitter transmits light from the first lens 130 and reflects light from the second lens 132 (e.g., via element 133) and combines the light.

The optical channels propagate though a common optical channel or lens 136/138 before being separated by a selection element 140 in a sensor package. The selection element 140 may be a spectral filter (e.g., a dichroic spectral filter) filtering and/or reflecting different wavelengths or colors of light from the differentiating element 134 to each of the first and second sensors 142/144. Alternatively, the selection element 140 may be a polarizer, such as a cube polarization beam splitter, reflecting half of the combined light (light 150a, 152a, 154a) while transmitting the other half of the combined light (light 150b, 152b,154b). As a result, two optical channels are imaged onto individual sensors for 3D imaging. In particular, the sensor package includes a first sensor 142 receiving light 150a, 152a, and 154a and a second sensor 144 receiving light 150b, 152b, and 154b. The first sensor 142 and second sensor 144 are oriented substantially perpendicular to, i.e., approximately ninety degrees from, from one another.

The lens system of FIG. 3 likewise advantageously provides a compact lens system. For example, the sensor package may be approximately 3.7 mm diagonal or less. Further, larger channel offset than the embodiment of FIG. 2 is possible. Resolution in the embodiment of FIG. 3 is on the order of 600 x 600 or more.

The invention further includes a method of generating three-dimensional video imagery. The method includes the steps of receiving light via a distal end of an imaging device, providing the light via an optical channel or path to one or more differentiating elements in the optical channel, and differentiating the light either spectrally or by polarization. The method further includes providing the differentiated light to a selection element (e.g., a spectral filter or polarizer), the selection element situated between first and second sensors at a proximal end of the optical channel, wherein the second sensor is oriented substantially perpendicular to the first sensor. The selection element receives the differentiated light and transmits a first portion of the light to the first sensor and a second portion of the light to the second sensor.

Although the invention has been described with reference to a particular arrangement of parts, features and the like, these are not intended to exhaust all possible arrangements or features, and indeed many modifications and variations will be ascertainable to those of skill in the art.

The present disclosure comprises embodiments according to the following clauses:
(1) An imaging device, comprising:
   an optical channel;
   a lens assembly, including one or more lenses, at a distal end of said optical channel;
   a differentiating element in said optical channel receiving light from said lens assembly and differentiating the light;
   a first sensor at a proximal end of said optical channel;
   a second sensor at the proximal end of said optical channel, said second sensor oriented substantially perpendicular to said first sensor; and
   a selection element between said first and second sensors receiving the differentiated light from said optical channel and transmitting a first portion of the differentiated light to said first sensor and a second portion of the differentiated light to said second sensor.
(2) The imaging device according to clause 1, wherein said differentiating element and said selection element are each spectral filters.
(3) The imaging device according to clause 1, wherein said differentiating element and said selection element are each polarizers.
(4) The imaging device according to anyone of clauses 1 to 3, further comprising a second lens assembly at the distal end of said optical channel, wherein the light is received via said lens assembly and the second lens assembly and combined by said differentiating element into said optical channel.
(5) The imaging device according to clause 4, wherein said differentiating element includes a cube polarization beam splitter.
(6) The imaging device according to clause 4, wherein said differentiating element includes a spectral filter.
(7) The imaging device according to clause 1, wherein said differentiating element includes two polarizers in a pupil plane of said optical channel receiving the light from said lens.
(8) The imaging device according to clause 7, wherein the first portion of the polarized light is received from a first one of the two polarizers and the second portion of the polarized light is received from a second one of the two polarizers.
(9) The imaging device according to anyone of clauses 1 to 8, wherein said first sensor, said second sensor and said selection element are comprised in a sensor package.
(10) The imaging device according to clause 9, wherein the sensor package has a diagonal dimension of 3.7 millimeters or less.
(11) The imaging device according to clause 9, wherein the sensor package has a diagonal dimension of less than 3 millimeters.
(12) The imaging device according to anyone of clauses 1 to 11, wherein the imaging device has a resolution of 600 x 600 or more.
(13) The imaging device according to anyone of clauses 1 to 11, wherein the imaging device has a resolution of 694 x 694 or more.
(14) An imaging device, comprising:
   a body;
   a shaft; and
   a lens system arranged within said shaft, the lens system including at least one lens adjacent to a distal end of said shaft, first and second sensors arranged substantially perpendicular to one another in said shaft, and a selection element between the first and second sensors in said shaft.
(15) The imaging device according to clause 14, wherein the selection element is a polarizer.
(16) The imaging device according to clause 14, wherein the selection element is a spectral filter.
(17) The imaging device according to anyone of clauses 14 to 16, said lens system further including a differentiating element in an optical path between the at least one lens and the selection element, the differentiating element receiving light from the at least one lens and transmitting differentiated light via the optical path to the selection element.
(18) The imaging device according to clause 17, the at least one lens including two lenses adjacent to the distal end of said shaft, each of the two lenses receiving light via the distal end of the imaging device.
(19) The imaging device according to clause 18, wherein the differentiating element is a cube polarization beam splitter combining the light from the two and transmitting polarized light to the selection element.
(20) The imaging device according to clause 18, wherein the differentiating element is a spectral filter.
(21) The imaging device according to anyone of clauses 14 to 20, wherein the first sensor, the second sensor and the selection element are comprised in a sensor package, wherein the sensor package has a diagonal dimension of 3.7 millimeters or less.
(22) The imaging device according to anyone of clauses 14 to 21, wherein the imaging device has a resolution of 600 x 600 or more.
(23) An imaging device, comprising:
   a first lens receiving light via a distal end of the imaging device;
   a second lens receiving light via a distal end of the imaging device;
   a first sensor and a second sensor, said second sensor oriented substantially perpendicular to said first sensor;
   a differentiating element receiving the light from said first lens and the light from said second lens, combining the light from the first lens with the light from said second lens into an optical channel, and transmitting differentiated light via the optical channel; and
   a selection element between said first and second sensors receiving the differentiated light via the optical channel and transmitting a first portion of the differentiated light to said first sensor and a second portion of the differentiated light to said second sensor.
(24) The imaging device according to clause 23, wherein said differentiating element and said selection element are each spectral filters.
(25) The imaging device according to clause 23, wherein said differentiating element and said selection element are each polarizers.

The present disclosure includes an imaging device, including an optical channel, a lens at a distal end of the optical channel, a differentiating element in the optical channel receiving light from the lens and differentiating the light into different wavelengths, a first sensor at a proximal end of the optical channel, a second sensor at the proximal end of the optical channel, the second sensor oriented substantially perpendicular to the first sensor, and a selection element between the first and second sensors receiving the differentiated light from the differentiating element via the optical channel and transmitting a first portion of the differentiated light to the first sensor and a second portion of the differentiated light to the second sensor.

## Claims

1. An imaging device (10), comprising:
an optical channel;
a lens assembly (30; 130), including one or more lenses, at a distal end of said optical channel;
**characterized by**
a differentiating element (31; 134) in said optical channel receiving light from said lens assembly (30; 130) and differentiating the light;
a first sensor (42; 142) at a proximal end of said optical channel;
a second sensor (44; 144) at the proximal end of said optical channel, said second sensor (44; 144) oriented substantially perpendicular to said first sensor (42; 142); and
a selection element (40; 140) between said first and second sensors receiving the differentiated light (50a, 52a, 54a, 50b, 52b, 54b; 150a, 152a, 154a, 150b, 152b, 154b) from said optical channel and transmitting a first portion (50a, 52a, 54a; 150a, 152a, 154a) of the differentiated light to said first sensor (42; 142) and a second portion (50b, 52b, 54b; 150b, 152b, 154b) of the differentiated light to said second sensor (44; 144).

2. The imaging device (10) according to claim 1, **characterized in that** said differentiating element (31; 134) and/or said selection element (40; 140) are each spectral filters or polarizers.

3. The imaging device (10) according to any one of the preceding claims, **characterized in that** the imaging device further comprises a second lens assembly (132) at the distal end of said optical channel, wherein the light is received via said lens assembly (130) and the second lens assembly (132) and combined by said differentiating element (134) into said optical channel.

4. The imaging device (10) according to claim 3, **characterized in that** said differentiating element (134) includes a cube polarization beam splitter or spectral filter.

5. The imaging device (10) according to any one of claims 1-2, **characterized in that** said differentiating element (31) includes two polarizers (32, 34) in a pupil plane of said optical channel receiving the light from said lens assembly (30).

6. The imaging device (10) according to claim 5, **characterized in that** the first portion (50a, 52a, 54a) of the polarized light is received from a first one (32) of the two polarizers and the second portion (50b, 52b, 54b) of the polarized light is received from a second one (34) of the two polarizers.

7. The imaging device (10) according to any one of the preceding claims, **characterized in that** said first sensor (42; 142), said second sensor (44; 144) and said selection element (40; 140) are comprised in a sensor package.

8. The imaging device (10) according to claim 7, **characterized in that** the sensor package has a diagonal dimension of 3.7 millimeters or less.

9. The imaging device (10) according to claim 7, **characterized in that** the sensor package has a diagonal dimension of less than 3 millimeters.

10. The imaging device (10) according to any one of the preceding claims, **characterized in that** the imaging device (10) has a resolution of 600 x 600 or more.

11. The imaging device (10) according to any one of the preceding claims, **characterized in that** the imaging device further comprises:
a body (12);
a shaft (14); and
a lens system arranged within said shaft (14), the lens system including said lens assembly (30; 130) adjacent to a distal end (20) of said shaft (14), said first and second sensors (42, 44; 142, 144) arranged substantially perpendicular to one another in said shaft (14), and said selection element (40; 140) between the first and second sensors (42, 44; 142, 144) in said shaft (14).

12. The imaging device (10) according to any one of the preceding claims, **characterized in that** the selection element (40; 140) is a polarizer or a spectral filter.

13. The imaging device (10) according to any one of the preceding claims, **characterized in that** said lens system further includes said differentiating element (31; 134) in an optical path between the lens assembly (30; 130) and the selection element (40; 140), the differentiating element (31; 134) receiving light from the lens assembly (30; 130) and transmitting differentiated light via the optical path to the selection element (40; 140).

14. The imaging device (10) according to any one of the preceding claims, **characterized in that**:
said lens assembly comprises two lenses adjacent to the distal end (20) of said shaft (14), each of the two lenses receiving light via the distal end of the imaging device, and
the differentiating element (31; 134) is either a cube polarization beam splitter combining the light from the two and transmitting polarized light to the selection element (40; 140) or a spectral filter.

15. An imaging device (10) according to any one of claims 3-4, **characterized in that**:
said lens assembly (130) comprises a first lens receiving light via a distal end of the imaging device (10);
said second lens assembly (132) comprises a second lens receiving light via a distal end of the imaging device (10);
said differentiating element (134) receiving the light from said first lens and the light from said second lens, combining the light from the first lens with the light from said second lens into the optical channel, and transmitting differentiated light via the optical channel; and
said selection element (140) between said first and second sensors (142, 144) receiving the differentiated light via the optical channel and transmitting the first portion (150a, 152a, 154a) of the differentiated light to said first sensor (142) and a second portion (150b, 152b, 154b) of the differentiated light to said second sensor (144).
